# EUROPEAN PATENT APPLICATION

(11) **EP 2 110 122 A2**
(43) Date of publication of application: **21.10.2009**
(21) Application number: 09005348.9
(22) Date of filing: 15.04.2009
(51) Int. Cl.: A61K 8/97, A61Q 19/00

(54) **Lipogels and ointments containing liquid or lyophilized aloe and other active prinpiples and extraction and aggregation process**

(30) Priority: 18.04.2008 IT SA20080008
(71) Applicant: Maffei, Aristide, 80040 Cercola (IT)
(72) Inventor: Maffei, Aristide, 80040 Cercola (IT)

(57) **Abstract**

This invention consists in the production of ointments and essential oils for cosmetic or therapeutical use and of essential oils for dietary use enriched with liquid or liophilized aloe and other plant extract. By an efficient method using mechanic tools and by the use of hydrostatic pressure, applied by a continuous dynamics, we have succeeded in enriching the oil used as base, for example jojoba oil, olive oil etc., with 15-30% of aloe, and scented with lavender flowers or many other different flowers participating, together with specially prepared myrrh resin, to the whole integrated extraction process, in order to obtain a special and innovative essential oil (lipogel). When ointments are to be prepared, this process is extended by the liophilization station, which consists of the lioliphilization of the concentrated aloe juice.

## Description

This invention consists of the production of ointments and essential oils for cosmetic or therapeutical use, and essential oils for dietary use, enriched with liquid or liophilized aloe and other plant extracts.

To fight the tendence of our skin to become lined, for healing wounds, lacerations, and skin abrasions, simple treatments can be used such as the application of oils and ointments having special beneficial effects. A quick, cheap and very efficient way to treat skin tissues is that of using some oils extracted from a variety of plants having healing, elasticizing or tonifying properties, directly applying them on the skin surface. If an edible oil, such as olive oil, enriched with active principles as aloe, is used as foundation according to this invention, it is also possible to use it as food having beneficial and therapeutical effects.

By an efficient method using a variety of mechanic tools and by the use of hydrostatic pressure, applied by a continuous dynamics, we have succeeded in enriching the oil used as base, for example jojoba oil, olive oil etc., with 15-30% of aloe, and scented with lavender flowers or many other different flowers participating, together with specially prepared myrrh resin, to the whole integrated extraction process, in order to obtain a special and innovative essential oil (lipogel). The latter is only an oily solon of molecules with apolar characteristics and, consequently, similar to those of oil; by using an essential oil (lipogel) the beneficial effects of oil can be increased and extended to the whole human body since the conveyance of active principles is made easy and fostered by the absorption of the main components of oil. This compound is obtained without using emulsifiers, without preparations of solvent gases etc., thus leaving all the properties of the single elements untouched.

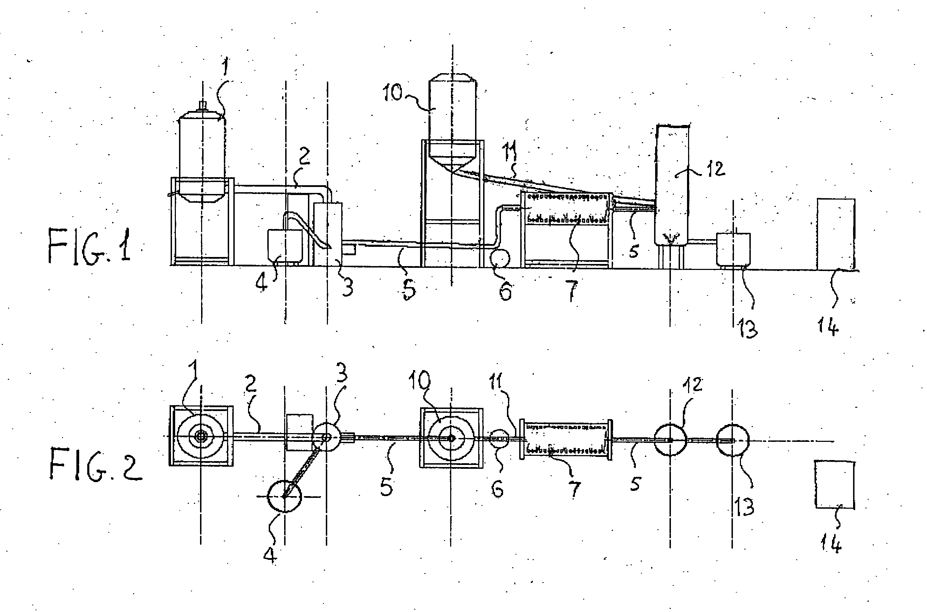

The process, fig. 1, and fig.2, to obtain an essential oil (lipogel) takes place essentially in the following way :
aloe leaves are put into the tank (1) where they are accurately washed with ozonized water, in order to eliminate any microbial charge, then its leaves are conveyed by a conveyor (2) into a centrifugal machine (3) within which the solid portion (pulp and fibre portions) are collected into a container (4) for other uses; the liquid (aloe juice), filtered and possibly added with antioxidant and/or fluidifying agents is canalized (59 and lifted by a pump (6) into the inox steel cylindrical chamber (7), where it is further concentrated and purified, next to a tank (10) which through a pipe (11) lets vegetal oil to be processed into the chamber (12) fall down by gravity in amounts at a speed compatible with the amount of aloe to be dispersed; the free surface of oil in the container undergoes an initial pressure of four and three atmospheres, but is graduated according to a continuous dynamics, which is so precise and such that within the expected period of time and according to the expected proportions, the liquid of aloe disperses homogeneously and irreversibly into the oil without any possibility for the oil, even if it udergoes a mixing process, to separate and participate into two different stages.

The finished product (lipogel) is then loaded into the addition container (13) ready to be added together with myrrh to other active principles and vegetal essences and scents.

When ointments are to be prepared, this process is extended by the liophilization station (14), which consists of the lioliphilization of the concentrated aloe juice that in this case is used pure and free from any antioxidant and/or fluidifying agents. Another oil which can be used as base is the jojoba oil (simmondsia chinensis), it has been used for hundreds of years by Indians as a universal remedy for treating the skin. It is called oil, but is a real "liquid wax". While all the other oils of seeds also contain glycerin in their molecule (triglycerides), jojoba oil does not contain glycerin and its molecule is linear, that is it is not branched, and that can explain its easy absorption by the skin and its power of penetrating through the little pores of the skin and the interstices of the derma. The properties of this oil are appreciated for its high degree of purity, the absence of any smell, for its resistance to warmth and growing rancid, besides the presence in it of natural antioxidants: tocopherols. Its use is recommended in all cases of early skin ageing, of dry skin, which is then more exposed to wrinkle lining because of its thinness. It is then possible, specially exporting the penetrating power of some components of the jojoba oil, to introduce into the skin tissues, important active principles which can be dissolved into oil thus producing an essential oil (lipogel).

The chemical-physical properties and characteristics of the jojoba oil can be well matched with many active principles contained in the aloe plant, and in order to implement the beneficial properties of the jojoba oil, an essential oil (lipogel) with aloe has been produced.

The aloe plant has been put in contact with the jojoba oil, which by an enrichment process, has been able to dissolve the main active components of this plant, such aloine and anthraquinonical compounds, which in literature have been proved as having beneficial effects on disorders and pathologies affecting the human body. The importance of obtaining an lipogel made from aloe is given by the easy penetration of active principles into the area of the body affected by a certain pathology and, by repeated applications over time, health conditions improve because of the penetration of active principles through the skin tissue. In addition to this, the topical application of oil on the skin, in general, has no contraindications, and this treatment can be carried out by a mere spreading or massage, which combines the relaxing action of the body with the opening of the pores fostering the penetration of the active components. The so obtained aloe essential oil combines the penetrating properties of the jojoba oil components with the well known beneficial effects of aloe; the synergy of effects helps to delay the skin ageing phenomenon and then the appearance of wrinkles and, in addition to this, the body is protected by the toxic effect of substances harmful for the body, because of the intervention of aloe and also myrrh components.

For the preparation of ointments it is possible to use simple oils, but it is more convenient to use one or more essential oil (lipogel) obtained by the above described process and among those listed below, besides jojoba oil:
Macadamia Oil (Terniflora)
Avocado oil (Persea gratissima)
Passionflower Oil (Ederlis Maracuja)
Oenothena oil (Oenothera biennis)

For the production of topical ointments for different skin stress pathologies, a basic formula can be the following for one hundred grams of product:
Myrrh, 2 gr.
Beeswax, 6 gr.
Liophilized aloe, 4 gr.
Castor oil, 20 gr.
Jojoba oil , 68 gr.

In the place of jojoba oil other oils among those listed above, can be used, but always in an amount of 68 gr. for every 100 gr. of ointment product.

The ointment can also contain in its formula more than one oil among the listed ones, but always in the whole proportion of 68% of weight.

Liophilized or dryed aloe is added in its oily phase because it solubilized oil protects and preserves it over time until it is applied on the skin; at this point a kind of solubilization intervenes due to the body water (sweat) evaporation which allows the absorption of aloe, which then releases its skin regenerating characteristics. We use liophilized or dryed aloe only in the ointments and not in creams just because ointments do not contain water or water in is not liquid state, which would irreparably damage liophilized aloe.

## Claims

1. Lipogels and ointments containing liquid or lyophilized aloe and other active principles **characterized in** the fact that the enriching of the oil used as base, for example jojoba oil, olive oil , Macadamia Oil , Avocado oil , Passionflower Oil, Oenothena oil with aloe is of 15-30% of aloe, and extraction process consisting in the fact that aloe leaves are put into the tank (1) where they are accurately washed with ozonized water, in order to eliminate any microbial charge, then its leaves are conveyed by a conveyor (2) into a centrifugal machine (3) within which the solid portion, pulp and fibre portions, are collected into a container (4) for other uses; the aloe liquid filtered and possibly added with antioxidant and/or fluidifying agents is canalized (5) and lifted by a pump (6) into the inox steel cylindrical chamber (7), where it is further concentrated and purified, next to a tank (10) which through a pipe (11) lets vegetal oil to be processed into the chamber (12) fall down by gravity in amounts at a speed compatible with the amount of aloe to be dispersed; the free surface of oil in the container undergoes an initial pressure of four and three atmospheres, but is graduated according to a continuous dynamics, which is such that within the expected period of time and according to the expected proportions, the liquid of aloe disperses homogeneously and irreversibly into the oil without any possibility for the oil, even if it udergoes a mixing process, to separate and participate into two different stages; the finished product, an essential oil or lipogel, is then loaded into the addition container (13) ready to be added together with myrrh to other active principles and vegetal essence and scents with lavender flowers or many other different flowers.

2. Lipogels and ointments containing liquid or lyophilized aloe and other active principles **characterized in that** for the preparation of ointments is to use one or more essential oil, or lipogel, among those listed below, besides jojoba oil, Macadamia oil, Avocado oil, Passionflower oil, Oenothena oil.

3. Lipogels and ointments containing liquid or lyophilized aloe and other active principles **characterized in that** for the production of topical ointments a basic formula can be the following for one hundred grams of product
Myrrh 2 gr.
Beeswax 6 gr.
Liophilized aloe 4 gr.
Castor oil 20 gr.
Jojoba oil 68 gr.

4. Lipogels and ointments containing liquid or lyophilized aloe and other active principles **characterized in that** lipogel can be used always in an amount of 68 gr for every 100 gr of ointment product or the ointment can also contain in its formula more than one oil but always in the whole proportion of 68% of weight.
